# EUROPEAN PATENT SPECIFICATION

(11) **EP 3 984 453 B1**
(45) Date of publication and mention of the grant of the patent: **01.10.2025**
(21) Application number: 20848138.2
(22) Date of filing: 18.03.2020
(51) Int. Cl.: A61B 5/145, A61B 5/00

(54) **METHOD FOR STABILIZING CONTINUOUS GLUCOSE MONITORING SYSTEM**
VERFAHREN ZUR STABILISIERUNG EINES KONTINUIERLICHEN GLUCOSEÜBERWACHUNGSSYSTEMS
PROCÉDÉ DE STABILISATION D'UN SYSTÈME DE SURVEILLANCE CONTINUE DU GLUCOSE

(30) Priority: 30.07.2019 KR 20190092492
(43) Date of publication of application: 20.04.2022
(73) Proprietor: i-Sens, Inc., Seoul 06646 (KR)
(72) Inventor: LEE, David, Seoul 06646 (KR); NAH, Ji Seon, Seoul 06646 (KR); SEO, Jung Hee, Seoul 06646 (KR); KANG, Young Jea, Seoul 06646 (KR)
(74) Representative: BCKIP Part mbB
(86) International application number: PCT/KR2020/003698
(87) International publication number: WO 2021/020688

(56) References cited:
- JP-A- 2016 501 070
- KR-A- 20180 064 854
- KR-B1- 101 760 899
- US-A1- 2014 135 605
- US-A1- 2015 316 559
- US-A1- 2017 079 564

## Description

### TECHNICAL FIELD

The present invention is generally related to a method for stabilizing a continuous blood glucose monitoring system, in more detail, a method for stabilizing a continuous blood glucose monitoring system for measuring biological information stably in a state that a sensor included in a sensor transmitter of the continuous blood glucose monitoring system is inserted to human body.

### BACKGROUND

Diabetes is a major cause of death and a cause of disability worldwide, and therefore, many people have health problems due to diabetes. Specially, diabetes is a serious disease that causes heart and kidney disease, blindness, nerve damage and high blood pressure. According to a long-term clinical study, the incidence of complications can be significantly reduced by appropriately managing blood glucose levels. Therefore, it is important to continuously manage diabetes, an important factor is self-monitoring of blood glucose levels.

In response to this demand, a self-diagnosis biometer in which a user can check a blood glucose level of the user by himself or herself has been widely distributed and used. A conventional blood glucose meter measures the blood glucose level of the user by putting the user's blood on a sensor strip, which is a test strip. Accordingly, the sensor strip with the blood is inserted into the blood glucose meter, and the blood glucose level measured through the sensor strip is displayed on the blood glucose meter.

At this time, the blood glucose meter receives an electrical signal generated by an electrochemical reaction between the collected blood and the reactant in the sensor strip, and measures the blood glucose level. Such a finger prick method helps diabetic patients to manage blood glucose, but it is difficult to accurately identify the blood glucose levels which are being frequently changed because it shows only the result at the time of the measurement.

Diabetics patients generally experience hyperglycemia and hypoglycemia, an emergency may occur in the hypoglycemic conditions, and the patients may become unconscious or die if a hypoglycemic condition lasts for an extended period of time without the supply of sugar. Accordingly, although rapid discovery of the hypoglycemic condition is critically important for diabetics, blood-collecting type glucose monitoring devices intermittently measuring glucose have limited ability to accurately measure blood glucose levels.

To overcome such a drawback, continuous glucose monitoring systems (CGMSs) inserted into the human body to measure a blood glucose level every few minutes have been developed, and therefore easily perform the management of diabetics and responses to an emergency situation.

The continuous blood glucose measurement system comprises a body attachable unit for measuring blood glucose by being inserted to human body and collecting test substances such as blood of the user, and a communication terminal for communicating with the body attachable unit and displaying the blood glucose level measured by the body attachable unit.

The sensor transmitter is inserted into the human body, and in a state that a part of a sensor included in the sensor transmitter is inserted to the human body, biological information is measured. At that time, due to the insertion of the sensor into the human body, the body can show the viral reaction by recognizing that foreign substance invades human body. If the viral reaction occurs in the human body, the measurement of the biological information measured through the sensor may not precisely performed.

Accordingly, before performing the measurement of the biological information, the conventional art waits for a certain time period for stabilization (e.g. two (2) hours) after inserting a sensor into the human body so that the sensor can be stably attached to the human body. During the stabilization time period, the sensor transmitter measures biological information of the human body, and the sensor transmitter transmits the measured biological information to the communication terminal. At that time, because the communication terminal does not output the measured data to the user for the stabilization time period of the sensor transmitter, the user is unable to check information regarding the biological information during the stabilization time period of the sensor transmitter.

The stabilization time period of the conventional art is set as a uniformly predetermined constant time (for example, two (2) hours). Therefore, even if the sensor transmitter is stabilized earlier than the uniformly predetermined constant time, the user cannot check the measured biological information data during the predetermined stabilization time period.

Furthermore, even if the stabilization time period lapses, the communication terminal displays the biological information data measured by the sensor transmitter even though the sensor transmitter is not stabilized during the predetermined stabilization time period, and therefore inaccurate biological information data can be provided to the user.

US 2014 / 0 135 605 A1 discloses a method for initializing an analyte sensor, such as a glucose sensor. Where a sensor has been disconnected and reconnected, a disconnection time is determined and a sensor initialization protocol is selected based upon the disconnection time. The sensor initialization protocol may include applying a first series of voltage pulses to the sensor. The method further includes a stabilization step in which a stabilization voltage is applied to the sensor for a first stabilization time. The method may further include determining whether the sensor is stable after applying the first stabilization voltage, and if the sensor is not stable, applying a second stabilization voltage to the sensor for a second stabilization time. Example stabilization time periods include times less than forty minutes, such as 10, 16, 20, and 26 minutes, or 30 minutes. The second stabilization time may be the same or different than the first stabilization time.

### DETAILED DESCRIPTION OF INVENTION

### Technical Problem

To solve the problem of the conventional art, the purpose of the present invention may be for providing a method for stabilizing a continuous blood glucose monitoring system which can variably adjust stabilization time set for stabilizing an output of biological information data measured by a sensor transmitter.

### Solution to Problem

To this end, the present invention provides a method in accordance with independent claim 1, preferred embodiments are defined by the dependent claims.

### Advantageous Effects of Invention

The present invention has advantageous technical effect in which accurate biological information data can be provided to the user by variably adjust stabilization time set for stabilizing an output of biological information data measured by a sensor transmitter depending on each of sensor transmitter.

Additionally, if the sensor transmitter is quickly stabilized, more accurate biological information can be provided to a user rapidly.

Further, a sensor transmitter which fails to be stabilized for a certain time period can be prevented to be used, and providing inaccurate biological information data to a user can be prevented by guiding the user to replace that sensor transmitter.

### BRIEF DESCRIPTION OF THE DRAWINGS

FIG. 1 is a schematic diagram for illustrating a continuous blood glucose measurement system.
FIG. 2 is a figure illustrating an applicator for attaching a sensor transmitter to a human body.
FIGS. 3 and 4 are figures for illustrating a process of attaching a sensor transmitter to a human body using an applicator.
FIG. 5 is a flowchart for illustrating a method for stabilizing a continuous blood glucose monitoring system according to an embodiment of the present invention.
FIG. 6 is a block diagram for illustrating a sensor transmitter of a continuous blood glucose measuring system.
FIG. 7 is a block diagram of a communication terminal of a continuous blood glucose monitoring system.
FIG. 8 is a figure for illustrating a case in which a sensor transmitter is quickly stabilized by a stabilization method of a continuous blood glucose monitoring system according to an embodiment.
FIG. 9 is a figure for illustrating a process for determining whether blood glucose information data is compromised in a stabilization method of a continuous blood glucose monitoring system according to an embodiment.
FIG. 10 is a figure for illustrating a case in which a stabilization time of a sensor transmitter is added in a stabilization method of a continuous blood glucose monitoring system.

### DESCRIPTION OF EMBODIMENTS

With reference to enclosed drawings, preferred embodiments are described in detail.

Also, it should be noted that the accompanying drawings are merely illustrative examples, whereas the invention is limited by the appended claims.

Hereinafter, with reference to the enclosed drawings, a method for stabilizing a continuous blood glucose monitoring system according to an embodiment is described in detail.

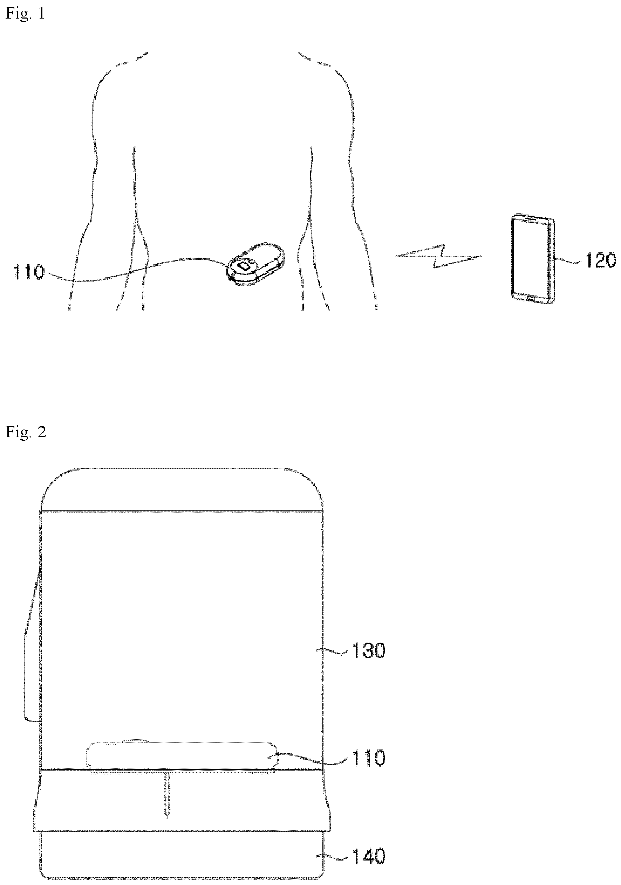

FIG. 1 is a schematic diagram for illustrating a continuous blood glucose measurement system according to an embodiment of the present disclosure.

Referring to FIG. 1, the continuous blood glucose measurement system according to an example of the present disclosure comprises a sensor transmitter (110) and a communication terminal (120).

The sensor transmitter (110) is attachable to human body and, when the sensor transmitter (110) is attached to the human body, an end portion of a sensor of the sensor transmitter (110) is inserted into skin to periodically extract body fluid of the human body and measure blood glucose.

The communication terminal (120) is a terminal configured to receive blood glucose information from the sensor transmitter (110) and output or display the received blood glucose information to a user, and for example, the communication terminal (120) may be a portable terminal (such as smartphone, tablet PC, or notebook and so on) configured to communicate with the sensor transmitter (110). However, the communication terminal (120) is not limited thereto, and may be any type of a terminal which has a communication function and program or application can be installed to.

The sensor transmitter (110) transmits the blood glucose information in response to request of the communication terminal (120) or at predetermined times periodically, and for data communication between the sensor transmitter (110) and the communication terminal (120), the sensor transmitter (110) and the communication terminal (120) are communicationally connected to each other over a wire by an USB cable and so on or communicationally connected in an wireless communication means such as infrared communication, NFC communication, Bluetooth, etc.

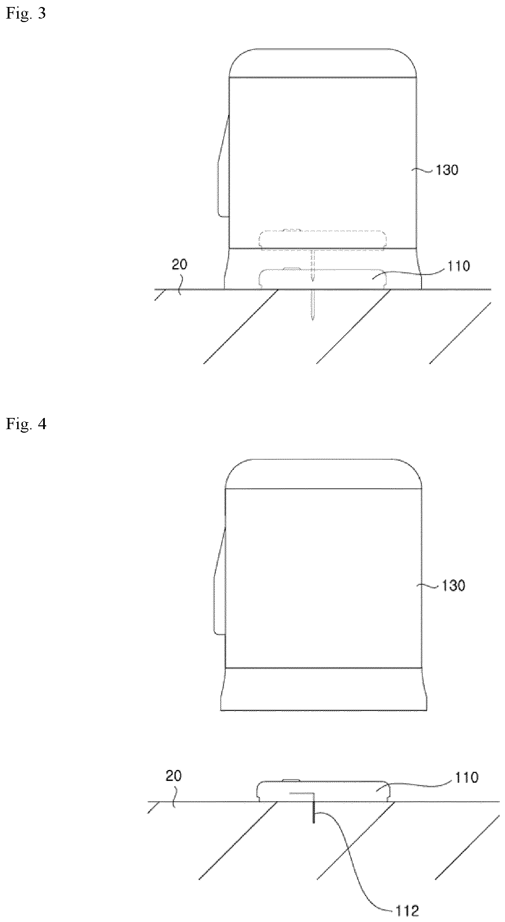

The sensor transmitter (110) is attached to a part of the human body by an applicator, FIG. 2 is a figure illustrating an applicator for attaching a sensor transmitter to a part of a human body according to an example of the present disclosure, and FIGS. 3 and 4 are figures for illustrating a process of attaching a sensor transmitter to a human body using an applicator according to an example of the present disclosure.

Firstly, an application (130) is now described by referring to FIG. 2, the sensor transmitter (110) is mounted in the applicator (130), and the applicator (130) can be operated so that the sensor transmitter (110) can be outwardly discharged to the outside of the applicator (130) by the manipulation of the user and then be attached to a specific portion of the human body of the user. The applicator (130) is formed to have a shape that one side of the applicator (130) is open, and the sensor transmitter (110) is installed to the applicator (130) through the open side of the applicator (130).

When the sensor transmitter (110) is attached to a part of the human body using the applicator (130), for inserting an end portion of the sensor included in the sensor transmitter (110) to skin, the applicator (130) comprises a needle (not shown) formed to cover the end portion of the sensor therein, a first elastic means (not shown) pushing the needle and the end portion of the sensor together towards the skin, and a second elastic means (not shown) configured to retract the needle only. The compressed state of the first elastic means (not shown) arranged to be compressed inside the applicator (130) by the configuration of the applicator (130) can be released, thereby inserting the needle and the end portion of the sensor simultaneously to the skin, and when the end portion of the sensor is inserted to the skin, the compressed state of the second elastic means (not shown) is released, thereby extracting the needle only. By the applicator (130), the user can safely and easily attach the sensor transmitter (110) to the skin.

A process of attaching the sensor transmitter (110) to the human body will be described in detail with reference to FIGS. 3 and 4, in a state that a protection cap (140) is separated or removed, an open side of the applicator (130) is closely placed on a specific part of skin (20) of the human body. When the applicator (130) is operated in a state that the applicator (130) is closely placed on the skin (20) of the human body, the sensor transmitter (110) is outwardly discharged from the applicator (130) and then attached to the skin (20). Here, an end portion of the sensor (112) is arranged to be exposed from the sensor transmitter (110) at a lower portion of the sensor transmitter (110), and a part of the end portion of the sensor (112) is inserted into the skin (20) by a needle installed at the applicator (130). Accordingly, the sensor transmitter (110) can be attached to the sensor (20) in a state that an end portion of the sensor (112) is inserted to the skin (20).

In the example of the present disclosure, an adhesive tape is provided at a surface of the sensor transmitter (110) contacting the human body so that the sensor transmitter (110) can be attached to the skin (20). Accordingly, if the applicator (130) is moved away from the skin (20) of the human body, the sensor transmitter (110) is fixedly attached to the skin (20) of the human body by the adhesive tape.

After that, if the power is supplied to the sensor transmitter (110), the sensor transmitter (110) is communicationally connected with the applicator (130), and the sensor transmitter (110) transmits the measured blood glucose information to the communication terminal.

The sensor transmitter (110) can measure not only the blood glucose information but also various biological information, and hereinafter blood glucose information is illustrated as one of examples of biological information.

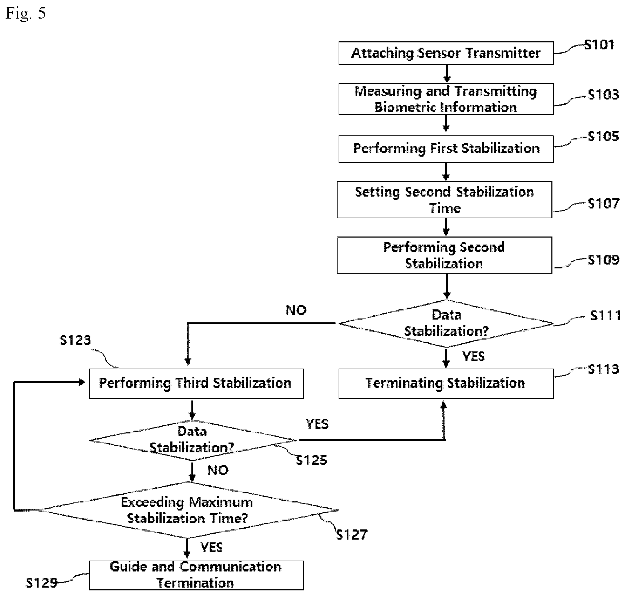

FIG. 5 is a flowchart for illustrating a method for stabilizing a continuous blood glucose monitoring system according to an embodiment of the present disclosure.

Referring to FIG. 5, a stabilization method of the sensor transmitter (110) of a continuous blood glucose monitoring system (100) is described. According to the present disclosure, the stabilization method of the sensor transmitter (110) performs the control of stabilization time at the communication terminal (120) based on blood glucose data measured by the sensor transmitter (110). Accordingly, the stabilization method is performed at the communication terminal (120).

Firstly, the sensor transmitter (110) is attached to human body (S101).

If the sensor transmitter (110) is attached to the human body, a part of a sensor included in the sensor transmitter (110) is inserted into the human body, and if the power is supplied to sensor transmitter (110), the sensor transmitter (110) is activated and performs communication connection with the communication terminal (120).

The sensor transmitter (110) performs the monitor or measurement of the blood glucose information of the human body, and the sensor transmitter (110) transmits the measured blood glucose information to the communication terminal (120) (S103).

If the sensor transmitter (110) is attached to the human body and activated, the measurement of the blood glucose information is performed from the time of being activated. And, the blood glucose information data measured by the sensor transmitter (110) is transmitted to the communication terminal (120).

If starting to receive the blood glucose information data from the sensor transmitter (110), the communication terminal (120) performs a first stabilization step (S105).

The first stabilization step is a process in which, until the blood glucose information data is stably measured, the communication terminal (120) does not output or display the received blood glucose information data to the user, but stores the received blood glucose information data. According to the present embodiment, the first stabilization step can be performed for a predetermined time period.

The communication terminal (120) sets time for a second stabilization step using the received blood glucose information data received from the sensor transmitter (110) while the first stabilization step is performed (S107).

The communication terminal (120) can adjust the time for the second stabilization step depending on whether the blood glucose information data received from the sensor transmitter (110) at the first stabilization stage is stable, if the blood glucose information data received from the sensor transmitter (110) at the first stabilization stage is stable, the time for the second stabilization stage can be set relatively short, and if the blood glucose information data received from the sensor transmitter (110) at the first stabilization stage is unstable, the time for the second stabilization stage can be set relatively long.

And, the communication terminal (120) performs the second stabilization stage according to the set time described above (S109).

Like the first stabilization stage, the second stabilization stage is a process in which the measured blood glucose information data is received from the sensor transmitter (110) and stored, but it is not outputted or displayed to the user.

As described above, when the second stabilization stage is performed for the set time, the communication terminal (120) checks whether the blood glucose information data measured and received from the sensor transmitter (110) is stabilized (S111).

At that step, if the communication terminal (120) determines that the blood glucose information data received from the sensor transmitter (110) is stabilized, the stabilization step of the sensor transmitter (110) terminates (S113). If the stabilization step of the sensor transmitter (110) terminates, the communication terminal (120) outputs or displays the blood glucose information data received from the sensor transmitter (110) after the stabilization step of the sensor transmitter (110) terminates so that the user can check the received blood glucose information data.

However, if the communication terminal (120) determines that the blood glucose information data received from the sensor transmitter (110) is not stabilized, the communication terminal (120) perform a third stabilization stage (S123).

Like the first stabilization stage and second stabilization stage, the third stabilization stage is a process in which the measured blood glucose information data is received from the sensor transmitter (110) and stored, but it is not outputted or displayed to the user.

And, while the third stabilization stage is being performed, the communication terminal (120) checks randomly (or at a predetermined time interval) whether the blood glucose information data received from the sensor transmitter (110) is stabilized (S125).

At this step, if the communication terminal (120) determines that the blood glucose information data is stabilized during the third stabilization stage, the communication terminal (120) moves to Step S113 to terminate the stabilization step of the sensor transmitter (110)a.

However, if the communication terminal (120) determines that the blood glucose information data received from the sensor transmitter (110) is not stabilized during the third stabilization stage, the communication terminal (120) check whether maximum stabilization time is reached (S127).

In the present disclosure, the stabilization process can be set so that the first to third stabilization steps can be performed within a predetermined time.

Accordingly, the communication terminal (120) can set maximum time for stabilization, and the maximum stabilization time means whole stabilization time for performing the first to third stabilization steps. For example, the communication terminal (120) may set the maximum time for the stabilization as three (3) hours, and if the first stabilization stage consumes one (1) hour and the second stabilization stage consumes for forty (40) minutes, then the third stabilization stage can be performed for one (1) hour and twenty (20) minutes. At the present step, the check of whether the maximum stabilization time is reached is checking whether three (3) hours which is the maximum time for the stabilization is reached.

Accordingly, if the maximum stabilization time is not reached, step S123 of the third stabilization stage continues to be performed.

And, when the maximum stabilization time is reached, the communication terminal (120) notifies to the user that the ongoing stabilization operation is not performed anymore and terminates the communication connection with that sensor transmitter (110) (S123).

At this step, the termination of the communication connection between the sensor transmitter (110) and the communication terminal (120) is a process of stopping the use of that sensor transmitter (110) because that sensor transmitter (110) cannot be used anymore.

Accordingly, because the blood glucose information data received from that sensor transmitter (110) cannot be stabilized during the stabilization time period, the communication terminal (120) terminates the communication with that sensor transmitter (110) not to use that sensor transmitter (110) which is incapable of stably measuring the blood glucose information data.

And, the communication terminal (120) displays a guide indicating that that sensor transmitter (110) cannot be used so that the user can check.

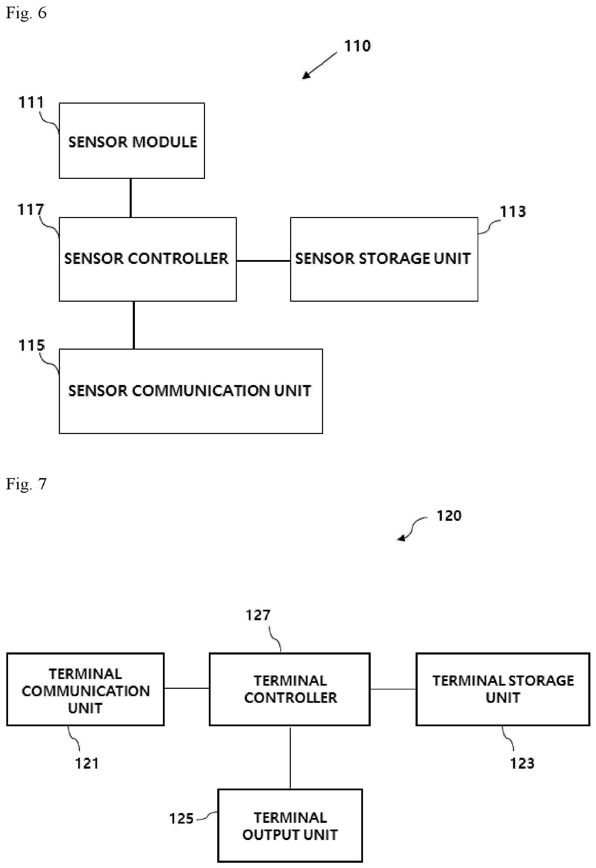

FIG. 6 is a block diagram for illustrating a sensor transmitter of a continuous blood glucose measuring system according to an example of the present disclosure.

According to an example of the present disclosure, referring to FIG. 6, the sensor transmitter (110) comprises a sensor module (111), a sensor storage unit (113), a sensor communication unit or communicator (115) and a sensor controller (117).

The sensor module (111) comprises a sensor (112), and a part of the sensor (112) is inserted into the human body to measure blood glucose information.

The sensor storage unit (113) stores data of the blood glucose information measured by the sensor module (111). At this embodiment, the blood glucose information data stored to the sensor storage unit (113) can be stored as digital signals.

The sensor communication unit (150) transmits the blood glucose information data stored to the sensor storage unit (113) to the communication terminal (120).

The sensor controller (117) controls the sensor module (111) to measure blood glucose information at a predetermined time interval. The sensor controller (117) transforms the blood glucose information data measured as analog signals into the digital signals, and the transformed digital signals of the blood glucose information data are stored to the storage unit. Additionally, the sensor controller (117) controls the sensor communication unit (115) to transmit the stored blood glucose information data to the communication terminal (120).

FIG. 7 is a block diagram of a communication terminal of a continuous blood glucose monitoring system according to an example of the present disclosure.

According to an example of the present disclosure, referring to FIG. 7, the communication terminal (120) includes a terminal communication unit (121), a terminal storage unit (123), a terminal output unit (125) and a terminal controller (127).

The terminal communication unit (121) communicates with the sensor communication unit (115) of the sensor transmitter (110), and receives the blood glucose information data from the sensor communication unit (115).

The terminal storage unit (123) stores the blood glucose information data received through the terminal communication unit (121).

The terminal output unit (125) outputs or displays the blood glucose information data received from the sensor transmitter (110) and stored to the terminal storage unit (123) so that the user can check the blood glucose information data. The terminal output unit (125) does not output or display the blood glucose information data being received from the sensor transmitter (110) during the stabilization operation, but outputs the blood glucose information data received from the sensor transmitter (110) after the completion of the stabilization operation.

The terminal controller (127) stores the blood glucose information data received through the terminal communication unit (121) to the terminal storage unit (123). And, the terminal controller (127) controls the terminal display unit (125) not to output or display the received blood glucose information data on the terminal display unit (125) during the stabilization operation, and performs only an operation of storing the blood glucose information data to the terminal storage unit (123). After the completion of the stabilization operation, the terminal controller (127) stores the blood glucose information data received from the sensor transmitter (110) to the terminal storage unit (123), and controls to output or display the blood glucose information data on the terminal output unit (125).

Further, the terminal controller (127) can adjust time of performing a stabilization operation of the sensor transmitter (110). The terminal controller (127) can adjust the time of performing the stabilization operation by controlling to perform stabilization for a time period set for the first stabilization stage, and setting stabilization time for the second stabilization stage and controlling to perform stabilization for the time period set for the second stabilization stage. And, the terminal controller (127) can control to perform the third stabilization stage until the maximum stabilization time is reached. As described above, the terminal controller (127) can terminate the stabilization operation in the middle of the third stabilization stage.

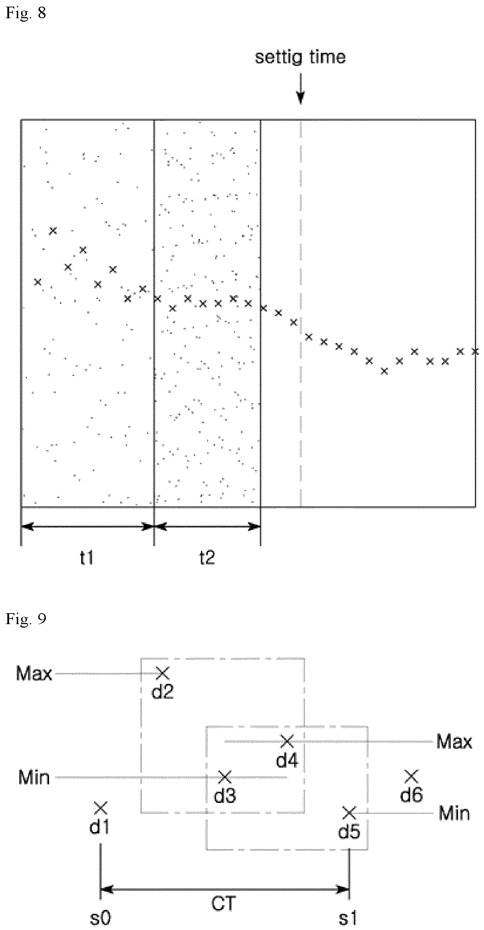

FIG. 8 is a figure for illustrating a case in which a sensor transmitter (110) is quickly stabilized by a stabilization method of a continuous blood glucose monitoring system according to an embodiment of the present invention, and FIG. 9 is a figure for illustrating a process for determining whether blood glucose information data is compromised in a stabilization method of a continuous blood glucose monitoring system according to an embodiment of the present disclosure.

Referring to FIG. 8, stabilization of blood glucose information data measured by the sensor transmitter (110) through the first stabilization stage and the second stabilization stage in the stabilization method of the present disclosure is described.

The communication terminal (120) can set that the first stabilization stage can be performed for a certain time period (t1). Accordingly, the first stabilization stage can be performed for a predetermined time period, for example, the first stabilization stage can be performed for one (1) hour.

While the first stabilization stage is being performed during the preset time period (t1), the communication terminal (120) checks whether the blood glucose information data received from the sensor transmitter (110) is within a set range. Alternatively, as described above, although the stabilization of the blood glucose information data can be checked using the blood glucose information data received by the communication terminal (120) during the set time period, but the present invention is not limited thereto, the stabilization of the blood glucose information data can be checked using at least a part of recent blood glucose information data among the received blood glucose information data.

By checking the blood glucose information data received from the sensor transmitter (110) during the first stabilization stage (t1), the communication terminal (120) can check whether the biological signal data received by the sensor transmitter (110) with respect to time is within a certain range. As illustrated, when the blood glucose information data received by the sensor transmitter (110) is within a certain range, the communication terminal (120) can set a time period that can be consumed at the second stabilization stage as being relatively short.

Referring to FIG. 9, an exemplary embodiment of checking stabilization of blood glucose information data at the first stabilization stage is described.

The communication terminal (120) can use recent blood glucose information data among the received blood glucose information data to check whether the blood glucose information data received from the sensor transmitter (110) is within a certain range. For example, while the first stabilization stage is being performed, the communication terminal (120) can check whether a difference between a maximum value (Max) and a minimum value (Min) of three (3) latest received blood glucose information data is within a set range.

Accordingly, when d1 to d4 of the blood glucose information data are sequentially received at the first stabilization stage, whether a difference between the maximum value and the minimum value of d2 to d4 is within a set range is checked. And, if d5 of the blood glucose information data is further received, whether a difference between the maximum value and the minimum value of d3 to d5 is within the set range is checked. If the difference between the maximum value and the minimum value of d3 to d5 is within the set range, using d6 of the blood glucose information data received next, whether a difference between the maximum value and the minimum value of d4 to d6 is within the set range is still within the set range is checked.

By checking most recently received blood glucose information data, whether the blood glucose information data can be compromised can be checked by checking whether the received blood glucose information data is within a set range while performing the first stabilization stage.

Additionally, the time of the second stabilization stage can be set using time (compromise time, CT) from a time point (s0) in which blood glucose information data is received first time to a time point (s1) in which a difference between a maximum value and a minimum value of five (5) latest received blood glucose information data is compromised within a set range. Accordingly, as the compromise time (CT) is shorter, the time of the second stabilization stage is shorter, and the compromise time (CT) is longer, the time of the second stabilization stage is longer.

In the present invention, during the time (t2) of performing the second stabilization stage, the communication terminal (120) sets a setting time, and controls to variably perform the second stabilization stage within the setting time. For example, the communication terminal (120) can set that the first stabilization stage can be performed within one (1) hour, and if the blood glucose information data received from the sensor transmitter (110) during the first stabilization stage is compromised within a certain range, the communication terminal (120) can control a time period for the second stabilization stage so that the second stabilization stage can be performed for a time period shorter than one (1) hour which is the setting time.

In this case, the communication terminal (120) can adjust time of performing the second stabilization stage according to a degree in which the blood glucose information data received from the sensor transmitter (110) is compromised to a certain range. Accordingly, the communication terminal (120) can control the time for performing the second stabilization stage so that, if the blood glucose information data received from the sensor transmitter (110) during the first stabilization stage is compromised quickly or compromised to a narrow range in proportion to a speed of being compromised to a certain range or a size of a compromised range, the time of performing the second stabilization stage can be set shorter.

And, the communication terminal (120) can control so that the time of performing the second stabilization stage does not exceed the setting time (or a reference time). For example, the communication terminal (120) can control so that the first stabilization stage can be performed for one (1) hour, a set time period, and the second stabilization stage can be performed for maximum one (1) hour, a set time period.

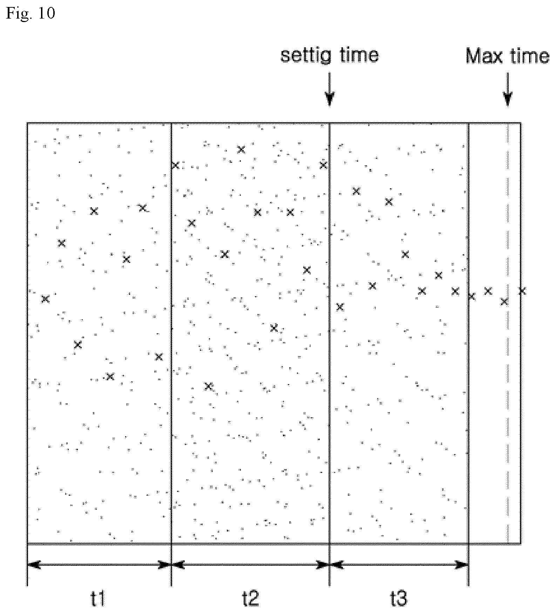

FIG. 10 is a figure for illustrating a case in which a stabilization time of a sensor transmitter is added in a stabilization method of a continuous blood glucose monitoring system.

Referring to FIG. 10, performing the stabilization of blood glucose information data measured by the sensor transmitter (110) through the first stabilization stage, the second stabilization stage and the third stabilization stage in the stabilization method of the present disclosure is described.

The communication terminal (120) can set that the first stabilization stage can be performed for a certain time period (t1). While the first stabilization stage is being performed, the communication terminal (120) checks whether the blood glucose information data received from the sensor transmitter (110) is compromised to a certain range, but as illustrated in FIG. 10, the blood glucose information data received from the sensor transmitter (110) is not comprised to the certain range. In this case, the communication terminal (120) can set time of the second stabilization stage so that the second stabilization stage can be performed until a setting time.

The time of performing the second stabilization stage is set by the communication terminal (120) as described above, and when the time consumed at the second stabilization stage is continued to the setting time, whether the blood glucose information data received from the sensor transmitter (110) is stabilized is checked. Of course, even though the second stabilization stage is not performed by the setting time, whether the blood glucose information data received from the sensor transmitter (110) is stabilized is checked.

At that time, if it is confirmed that the blood glucose information data received from the sensor transmitter (110) during the second stabilization stage is stabilized so as to be compromised to a certain range, the whole stabilization operation terminates. However, as illustrated in FIG. 10, if the stabilization so as to be compromised to a certain range does not occur, the third stabilization stage is performed.

While the third stabilization stage is being performed, the communication terminal (120) continuously checks whether the blood glucose information data received from the sensor transmitter (110) is stabilized. When the blood glucose information data received from the sensor transmitter (110) is stabilized in the middle of the third stabilization stage, the third stabilization stage terminates, and the whole stabilization operation can terminate.

When the third stabilization stage terminates, the communication terminal (120) terminates the whole stabilization operation of the sensor transmitter (110), and after the whole stabilization operation terminates, the communication terminal (120) outputs the blood glucose information data received from the sensor transmitter (110) through the terminal output (125).

However, when the stabilization of the blood glucose information data received from the sensor transmitter (110) is not completed during the third stabilization stage, the communication terminal (120) can control to perform the third stabilization stage until the maximum stabilization time Max time. By continuing to perform the third stabilization stage until the Max time, if the blood glucose information data received from the sensor transmitter (110) is stabilized, the whole stabilization operation terminates.

If the blood glucose information data received from the sensor transmitter (110) is not stabilized even though the third stabilization stage is performed until the maximum stabilization time, the communication terminal (120) determines that that sensor transmitter (110) is defective and cannot be used anymore, and immediately terminates the communication connection with that sensor transmitter (110). Alternatively, the communication terminal (120) can output or display, on the terminal output unit (125), a guide for checking whether or not the communication connection with that sensor transmitter (110) terminates to the user. When a request for terminating the communication connection between that sensor transmitter (110) and the communication terminal (120) is inputted from the user, the communication terminal (120) terminates the communication connection with that sensor transmitter (110).

If necessary, after the termination of the communication connection with the sensor transmitter (110), the blood glucose information data received through the stabilization operation from the sensor transmitter (110) can be outputted on the terminal output unit (125).

Here, if necessary, the communication terminal (120) can store information of an identifier and a serial number of the sensor transmitter (110) of which blood glucose information data is not stabilized. And, the communication terminal (120) can transmit that information to a server and so on so that the sensor transmitter (110) having that identifier and that serial number cannot be used afterward.

Additionally, as described above, if the blood glucose information data measured by the sensor transmitter (110) is not stabilized, the communication terminal (120) controls to display a guide for providing a reward for that sensor transmitter (110) to the user.

As described above, the foregoing detailed descriptions regarding the present invention have been presented by way of exemplary embodiments, but the detailed exemplary embodiments are presented as preferred examples, and therefore it should be understood that the present invention shall not be limited to those exemplary embodiments, and the scope of the present invention shall be defined by the appended claims.

## Claims

1. A stabilization method comprising:
receiving, by a communication terminal (120), biological information data transmitted from a sensor transmitter (110), wherein the sensor transmitter (110) is configured to be attachable to a body part of a user and measure biological information;
performing a first stabilization step in which the communication terminal (120) checks stabilization of the biological information data received from the sensor transmitter (110) during a first time period set by the communication terminal (120);
variably setting, by the communication terminal (120), a second time period for a second stabilization step based on the biological information data transmitted from the sensor transmitter (110) during the first time period;
performing, for the second time period, a second stabilization step in which the communication terminal (120) checks stabilization of the biological information data received from the sensor transmitter (110) during the second time period; and
terminating (S113) the second stabilization step if the biological information data is determined to be stabilized.

2. The stabilization method of claim 1, wherein the second time period is set according to a degree in which the biological information data received from the sensor transmitter (110) during the first time period is compromised to a certain range.

3. The stabilization method of claim 1 or 2, further comprising:
displaying the biological information data received from the sensor transmitter (110) so that the user is able to check the biological information data after the second stabilization step is completed.

4. The stabilization method of any one of the preceding claims, wherein the checking of whether the biological information data is stabilized is performed based on whether the biological information data received from the sensor transmitter (110) is compromised to a set range.

5. The stabilization method of any one of the preceding claims, further comprising:
performing a third stabilization step in which the communication terminal (120) checks whether stabilization of the biological information data received from the sensor transmitter (110) by the communication terminal (120) during a third time period, if the biological information data received from the sensor transmitter (110) is not stabilized while performing the second stabilization step.

6. The stabilization method of claim 5, wherein, if the biological information data received from the sensor transmitter (110) is stabilized while performing the third stabilization step, the communication terminal (120) terminates the third stabilization step.

7. The stabilization method of claim 6, wherein the third time period is determined such that third stabilization step is performed not to exceed a maximum time for performing the first to third stabilization steps.

8. The stabilization method of claim 7, further comprising, if it is determined that the biological information data received from the sensor transmitter (110) has not stabilized until the third time period elapses terminating, by the communication terminal (120), communication connection with the sensor transmitter (110).

9. The stabilization method of claim 8, further comprising, when the communication terminal (120) terminates the communication connection with the sensor transmitter (110), outputting, by the communication terminal (120), an interface for receiving, from the user, confirmation regarding termination of the communication connection with the sensor transmitter (110).

10. The stabilization method of claim 8 or 9, further comprising, if the communication terminal (120) terminates the communication connection with the sensor transmitter (110), displaying, on the communication terminal (120), a guide indicating that the sensor transmitter (110) cannot be used.

## Patentansprüche

1. Stabilisierungsverfahren, umfassend:
Empfangen, durch ein Kommunikationsendgerät (120), von biologischen Informationsdaten, die von einem Sensortransmitter (110) übertragen werden, wobei der Sensortransmitter (110) dazu eingerichtet ist, um an einem Körperteil eines Benutzers angebracht zu werden und biologische Informationen zu messen;
Durchführen eines ersten Stabilisierungsschritts, in dem das Kommunikationsendgerät (120) eine Stabilisierung der biologischen Informationsdaten, die von dem Sensortransmitter (110) empfangen werden, während eines ersten Zeitraums, der durch das Kommunikationsendgerät (120) eingestellt wird, überprüft;
variables Einstellen, durch das Kommunikationsendgerät (120), eines zweiten Zeitraums für einen zweiten Stabilisierungsschritt basierend auf den biologischen Informationsdaten, die von dem Sensortransmitter (110) während des ersten Zeitraums übertragen werden;
Durchführen, für den zweiten Zeitraum, eines zweiten Stabilisierungsschritts, in dem das Kommunikationsendgerät (120) eine Stabilisierung der biologischen Informationsdaten, die von dem Sensortransmitter (110) während des zweiten Zeitraums empfangen werden, überprüft; und
Beenden (S113) des zweiten Stabilisierungsschritts, wenn bestimmt wird, dass die biologischen Informationsdaten stabilisiert sind.

2. Stabilisierungsverfahren nach Anspruch 1, wobei der zweite Zeitraum gemäß einem Grad eingestellt wird, in dem die biologischen Informationsdaten, die von dem Sensortransmitter (110) während des ersten Zeitraums empfangen werden, auf einen bestimmten Bereich beeinträchtigt sind.

3. Stabilisierungsverfahren nach Anspruch 1 oder 2, ferner umfassend:
Anzeigen der biologischen Informationsdaten, die von dem Sensortransmitter (110) empfangen werden, so dass der Benutzer in der Lage ist, die biologischen Informationsdaten zu überprüfen, nachdem der zweite Stabilisierungsschritt abgeschlossen ist.

4. Stabilisierungsverfahren nach einem der vorhergehenden Ansprüche, wobei das Überprüfen, ob die biologischen Informationsdaten stabilisiert sind, basierend darauf durchgeführt wird, ob die biologischen Informationsdaten, die von dem Sensortransmitter (110) empfangen werden, auf einen eingestellten Bereich beeinträchtigt sind.

5. Stabilisierungsverfahren nach einem der vorhergehenden Ansprüche, ferner umfassend:
Durchführen eines dritten Stabilisierungsschritts, in dem das
Kommunikationsendgerät (120) überprüft, ob eine Stabilisierung der biologischen Informationsdaten, die von dem Sensortransmitter (110) durch das Kommunikationsendgerät (120) während eines dritten Zeitraums empfangen werden, vorliegt, wenn die biologischen Informationsdaten, die von dem Sensortransmitter (110) empfangen werden, nicht stabilisiert sind, während der zweite Stabilisierungsschritt durchgeführt wird.

6. Stabilisierungsverfahren nach Anspruch 5, wobei, wenn die biologischen Informationsdaten, die von dem Sensortransmitter (110) empfangen werden, stabilisiert sind, während der dritte Stabilisierungsschritt durchgeführt wird, das Kommunikationsendgerät (120) den dritten Stabilisierungsschritt beendet.

7. Stabilisierungsverfahren nach Anspruch 6, wobei der dritte Zeitraum derart bestimmt wird, dass der dritte Stabilisierungsschritt durchgeführt wird, um eine maximale Zeit zum Durchführen des ersten bis dritten Stabilisierungsschritts nicht zu überschreiten.

8. Stabilisierungsverfahren nach Anspruch 7, ferner umfassend, wenn bestimmt wird, dass die biologischen Informationsdaten, die von dem Sensortransmitter (110) empfangen werden, nicht stabilisiert sind, bis der dritte Zeitraum abläuft, Beenden, durch das Kommunikationsendgerät (120), einer Kommunikationsverbindung mit dem Sensortransmitter (110).

9. Stabilisierungsverfahren nach Anspruch 8, ferner umfassend, wenn das Kommunikationsendgerät (120) die Kommunikationsverbindung mit dem Sensortransmitter (110) beendet, Ausgeben, durch das Kommunikationsendgerät (120), einer Schnittstelle zum Empfangen, von dem Benutzer, einer Bestätigung bezüglich einer Beendigung der Kommunikationsverbindung mit dem Sensortransmitter (110).

10. Stabilisierungsverfahren nach Anspruch 8 oder 9, ferner umfassend, wenn das Kommunikationsendgerät (120) die Kommunikationsverbindung mit dem Sensortransmitter (110) beendet, Anzeigen, auf dem Kommunikationsendgerät (120), einer Anleitung, die angibt, dass der Sensortransmitter (110) nicht verwendet werden kann.

## Revendications

1. Procédé de stabilisation, comprenant :
la réception, par un terminal de communication (120), de données d'informations biométriques transmises par un émetteur de détection (110), ledit émetteur de détection (110) étant configuré pour pouvoir être fixé à une partie du corps d'un utilisateur et mesurer des informations biométriques ;
l'exécution d'une première phase de stabilisation lors de laquelle le terminal de communication (120) contrôle la stabilisation des données d'informations biométriques reçues de l'émetteur de détection (110) pendant une première période définie par le terminal de communication (120) ;
la définition variable, par le terminal de communication (120), d'une deuxième période pour une deuxième phase de stabilisation sur la base des données d'informations biométriques transmises par l'émetteur de détection (110) pendant la première période;
l'exécution, pour la deuxième période, d'une deuxième phase de stabilisation où le terminal de communication (120) contrôle la stabilisation des données d'informations biométriques reçues de l'émetteur de détection (110) pendant la deuxième période ; et
la cessation (S113) de la deuxième phase de stabilisation s'il est déterminé que les données d'informations biométriques sont stabilisées.

2. Procédé de stabilisation selon la revendication 1, où la deuxième période est définie suivant un degré où les données d'informations biométriques reçues de l'émetteur de détection (110) pendant la première période sont altérées dans une certaine mesure.

3. Procédé de stabilisation selon la revendication 1 ou la revendication 2, comprenant en outre :
l'affichage des données d'informations biométriques reçues de l'émetteur de détection (110), permettant à l'utilisateur de contrôler les données d'informations biométriques à l'issue de la deuxième phase de stabilisation.

4. Procédé de stabilisation selon l'une des revendications précédentes, où le contrôle si les données d'informations biométriques sont stabilisées est effectué en fonction de l'altération dans une certaine mesure des données d'informations biométriques reçues de l'émetteur de détection (110).

5. Procédé de stabilisation selon l'une des revendications précédentes, comprenant en outre :
l'exécution d'une troisième phase de stabilisation lors de laquelle le terminal de communication (120) contrôle la stabilisation des données d'informations biométriques reçues de l'émetteur de détection (110) par le terminal de communication (120) pendant une troisième période, si les données d'informations biométriques reçues de l'émetteur de détection (110) ne sont pas stabilisées pendant l'exécution de la deuxième phase de stabilisation.

6. Procédé de stabilisation selon la revendication 5, où, si les données d'informations biométriques reçues du capteur émetteur (110) sont stabilisées pendant l'exécution de la troisième phase de stabilisation, le terminal de communication (120) met fin à la troisième phase de stabilisation.

7. Procédé de stabilisation selon la revendication 6, où la troisième période est déterminée de sorte que la troisième phase de stabilisation n'excède pas un temps maximum pour l'exécution des trois phases de stabilisation.

8. Procédé de stabilisation selon la revendication 7, comprenant en outre, s'il est déterminé que les données d'informations biométriques reçues de l'émetteur de détection (110) ne sont pas stabilisées jusqu'à ce que la troisième période se soit écoulée, une connexion de communication par le terminal de communication (120) avec l'émetteur de détection (110).

9. Procédé de stabilisation selon la revendication 8, comprenant en outre, lorsque le terminal de communication (120) met fin à la connexion de communication avec l'émetteur de détection (110), la génération, par le terminal de communication (120), d'une interface pour recevoir, de l'utilisateur, une confirmation relative à la cessation de la connexion de communication avec l'émetteur de détection (110).

10. Procédé de stabilisation selon la revendication 8 ou la revendication 9, comprenant en outre, si le terminal de communication (120) met fin à la connexion de communication avec l'émetteur de détection (110), l'affichage, sur le terminal de communication (120), d'un guide indiquant que l'émetteur de détection (110) ne peut pas être utilisé.
